# EUROPÄISCHE PATENTANMELDUNG

(11) **EP 0 648 447 A1**
(43) Veröffentlichungstag der Anmeldung: **19.04.1995**
(21) Anmeldenummer: 94110628.8
(22) Anmeldetag: 07.07.1994
(51) Int. Cl.: A45D 33/00, A45D 33/04, A45D 33/36, A45D 40/26

(54) **Vorrichtung zum Auftragen von in granulatförmiger Form vorliegendem lösbarem Körperpflegemittel**

(30) Priorität: 19.10.1993 DE 4335633
(71) Anmelder: Erlhöfer, Dieter, D-58566 Kierspe (DE)
(72) Erfinder: Erlhöfer, Dieter, D-58566 Kierspe (DE)
(74) Vertreter: Schmidt, Steffen J., Dipl.-Ing.

(57) **Zusammenfassung**

Eine Vorrichtung zum Auftragen von granulatförmigem Körperpflegemittel (12), wie insbesondere Alaungranulat, weist einen Behälter (10) auf zur Aufnahme des Granulats (12). Ein Tuch (14) ist am Behälter (10) so befestigt, daß es das granulatförmige Material so abdeckt, daß es anfeuchtbar ist. Durch Befeuchtung gelöstes Körperpflegemittel kann durch das Tuch zum Auftragen auf die Haut durchtreten.

## Beschreibung

Die Erfindung betrifft eine Vorrichtung zum Auftragen von in granulatförmiger Form vorliegendem lösbarem Körperpflegemittel, insbesondere Alaungranulat mit einem Behälter zur Aufnahme des Körperpflegemittels und einer Auftragsvorrichtung, über die das Körperpflegemittel auf die Haut auftragbar ist.

Seit langem sind Alaunsteine als Körperpflegemittel bekannt. Zum Beispiel werden kristallförmige Alaunsteine als Deodorant verwendet. Auch zum Blutstillen bei kleineren Wunden (z. B. beim Rasieren) finden Alaunsteine Verwendung.

Dabei wird der Alaunstein mit Wasser angefeuchtet, so daß sich etwas schwefelsaure Tonerde löst, welche auf die Haut aufgetragen wird. Es bildet sich ein reizfreier Säureschutzmantel, der Bakterienwachstum behindert und somit, bei Anwendung als Deodorant, Körpergeruch verhindert.

Wird gemäß dem Stand der Technik der Alaunstein bei Benutzung mit Wasser angefeuchtet, so wird er dadurch glitschig. Infolgedessen kann es beim Gebrauch dazu kommen, daß der Stein zu Boden fällt und in kleine Brocken zerspringt und somit nicht mehr zu gebrauchen ist.
Ein weiterer Nachteil des herkömmlichen Gebrauchs von Alaunsteinen liegt darin, daß beim Gebrauch des Steins Splitter abspringen können und sich dadurch scharfe Kanten bilden, die störend oder sogar verletzend wirken können.

Aus dem DE G 93 10 962.8 U1 ist eine Spenderflasche zum Auftragen eines in granulatförmiger Form vorliegendes lösbares Körperpflegemittel, insbesondere Alaungranulat, bekannt, die einen Behälter zur Aufnahme des Körperpflegemittels und eine Auftragsvorrichtung aufweist. Durch die Auftragsvorrichtung kann das Körperpflegemittel auf die Haut aufgetragen werden. Bei diesem Stand der Technik ist die Auftragsvorrichtung entweder als Rollerkugel, als Pumpzerstäuber oder als Dosierverschluß ausgebildet. Diese Ausgestaltung bedingt, daß das Körperpflegemittel in dem Behälter bereits in zumindest teilweise gelöster Form vorliegen muß, damit es auf die Haut auftragbar ist. Dies hat zur Folge, daß der Behälter einen flüssigkeitsdichten Verschluß benötigt und außerdem groß genug sein muß, um in ausreichender Menge neben dem Körperpflegemittel das zu dessen Lösung erforderliche Lösungsmittel aufzunehmen. Dies ist insbesondere für die Verwendung eines derartigen Körperpflegemittels auf Reisen problematisch, da bei einem Zerbrechen des Behälters oder einem undichten Verschluß der Inhalt des Behälters sich in einer Reisetasche sich entleeren kann. Darüber hinaus ist diese Anordnung wegen des in dem Behälter aufzunehmenden Lösungsmittels zwangsläufig relativ großbauend.

Aus der FR-PS-1 524 192 ist eine Vorrichtung zum Auftragen von Puder bekannt, bei der in einer zylindrischen Hülse eine federbelastete Bodenplatte aufgenommen ist, die den Puder in Richtung einer Rollauftragvorrichtung drängt.

Der Erfindung liegt die Aufgabe zugrunde, die eingangs genannte Vorrichtung dahingehend zu verbessern, daß sie einfach und sicher handhabbar ist und das Körperpflegemittel nicht notwendig in großkristalliner Form als massiver, einstückiger Stein vorliegen muß.

Die erfindungsgemäße Vorrichtung sieht vor, daß der Behälter an zumindest einer Seite durch eine als Tuch ausgebildete Auftragsvorrichtung abgedeckt ist, welches das granulatförmige Körperpflegemittel so abdeckt, daß es bei Gebrauch kurzzeitig mit z. B. Wasser anfeuchtbar ist, so daß sich etwas Körperpflegemittel von der festen Phase löst und durch das Tuch auf die menschliche Haut auftragbar ist.

Eine bevorzugte Ausgestaltung der erfindungsgemäßen Vorrichtung sieht vor, daß eine Feder am Behälter abgestützt ist, die das Körperpflegemittel gegen das Tuch drückt, so daß das Granulat und das Tuch eine ballige, pralle Form bilden und das Körperpflegemittel großflächig auf die Haut aufbringbar ist.

Eine weitere bevorzugte Ausgestaltung der Vorrichtung sieht vor, daß ein in bezug auf den Behälter verschiebliches Innenteil im Behälter angeordnet ist, das auf einer Seite von der Feder beaufschlagt ist und auf der anderen Seite das Körperpflegemittel gegen das Tuch drückt.

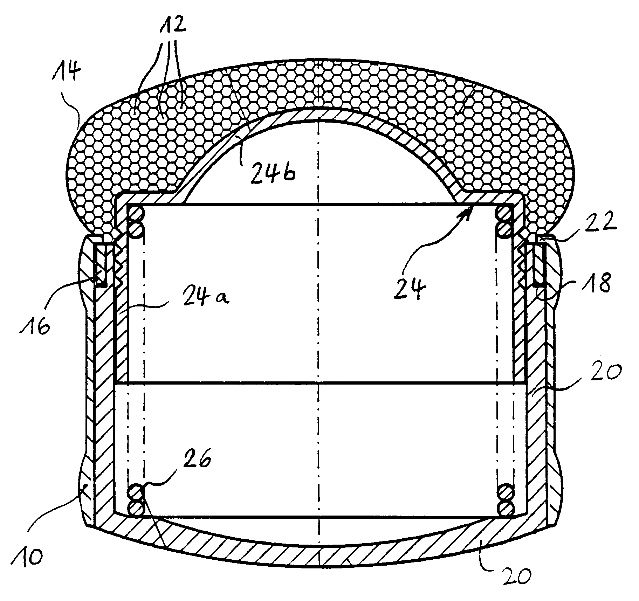

Nachfolgend wird ein Ausführungsbeispiel der Erfindung anhand der Zeichnung näher erläutert. Die Figur zeigt teilweise im Schnitt eine Vorrichtung zum Auftragen von granulatförmigem Körperpflegemittel.

Die Vorrichtung weist einen Behälter 10 aus Kunststoff auf. Der Behälter ist beim dargestellten Ausführungsbeispiel zylinderförmig. Auf einer offenen Stirnseite des Behälters 10 überspannt ein Tuch 14 granulatförmige Alaun-Kristalle mit einer Korngröße im Bereich von 10 mm oder kleiner. Die als Granulat vorliegenden Alaunkristalle 12 werden also durch das Tuch 14 kissenartig eingeschlossen. Das Tuch 14 stülpt sich aus dem Behälter 10 pilzartig vor. Das Tuch 14 kann aus jedem Material gefertigt sein, das eine Befeuchtung des Granulats zuläßt und angelöstes Alaun durchläßt.

Zur Befestigung ist das Tuch 14 in einem Ring 16 eingespannt.

In dem Behälter 10 ist ein Einsatz 20 eingesetzt, dessen Außenkontur etwa der Innenkontur des Behälters 10 entspricht. Der Einsatz 20 weist an seinem oberen Ende einen umlaufenden Absatz 18 auf, in den der Ring 16 paßgenau eingesetzt ist. Das Tuch 14 wird zwischen dem Ring 18 und dem Einsatz 20 befestigt, beispielsweise durch Verklebung oder auch Anschmelzen des Materials.

Der Behälter 10 umgreift mit einer Nase 22 den Ring 16.

In den Einsatz 20 ist ein Innenteil 24 eingesetzt, daß einen zylinderförmigen Abschnitt 24a und einen stirnseitigen ausgebauchten Abschnitt 24b aufweist. Mit dem ausgebauchten Abschnitt 24b greift das Innenteil 24 von innen in das Granulat-Kissen. Eine Feder 26 stützt sich mit einem Ende am Einsatz 20 ab und drückt mit dem anderen Ende das Innenteil 24 in Richtung auf das Tuch 14 und sorgt somit für eine pralle Ausbauchung des aus dem Granulat und dem Tuch 14 gebildeten Kissens, so daß eine ballige, prall gefüllte Auswölbung entsteht.

## Patentansprüche

1. Vorrichtung zum Auftragen von in granulatförmiger Form vorliegenden lösbaren Körperpflegemittel (12), insbesondere Alaungranulat, mit einem Behälter (10) zur Aufnahme des Körperpflegemittels und einer Auftragsvorrichtung (14), über die das Körperpflegemittel (12) auf die Haut auftragbar ist, dadurch **gekennzeichnet**, daß die Auftragsvorrichtung als Tuch (14) ausgebildet ist, das das granulatförmige Körperpflegemittel (12) abdeckt.

2. Vorrichtung nach Anspruch 1, dadurch **gekennzeichnet**, daß eine Feder (26) am Behälter (10) abgestützt ist, die das Körperpflegemittel gegen das Tuch (14) drückt.

3. Vorrichtung nach Anspruch 2, dadurch **gekennzeichnet**, daß ein in bezug auf den Behälter (10) verschiebliches Innenteil (24) im Behälter (10) angeordnet ist, das auf einer Seite von der Feder (22) beaufschlagt ist und auf der anderen Seite das Körperpflegemittel (12) gegen das Tuch (14) drückt.
